# EUROPEAN PATENT SPECIFICATION

(11) **EP 3 777 810 B1**
(45) Date of publication and mention of the grant of the patent: **26.11.2025**
(21) Application number: 19908549.9
(22) Date of filing: 20.12.2019
(51) Int. Cl.: G06F 3/01, G02B 27/01, G06F 3/0346, G06F 3/147

(54) **REHABILITATION ASSISTANCE DEVICE, REHABILITATION ASSISTANCE SYSTEM, REHABILITATION ASSISTANCE METHOD, AND REHABILITATION ASSISTANCE PROGRAM**
REHABILITATIONSUNTERSTÜTZUNGSVORRICHTUNG, REHABILITATIONSUNTERSTÜTZUNGSSYSTEM, REHABILITATIONSUNTERSTÜTZUNGSVERFAHREN UND REHABILITATIONSUNTERSTÜTZUNGSPROGRAMM
DISPOSITIF D'AIDE À LA RÉÉDUCATION, SYSTÈME D'AIDE À LA RÉÉDUCATION, PROCÉDÉ D'AIDE À LA RÉÉDUCATION, ET PROGRAMME D'AIDE À LA RÉÉDUCATION

(30) Priority: 07.01.2019 JP 2019000904; 26.04.2019 JP 2019085585
(43) Date of publication of application: 17.02.2021
(73) Proprietor: Medivr, Inc., Toyonaka City, Osaka 561-0872 (JP)
(72) Inventor: HARA Masahiko, Toyonaka City, Osaka 5610872 (JP)
(74) Representative: Betten & Resch
(86) International application number: PCT/JP2019/050118
(87) International publication number: WO 2020/145092

(56) References cited:
- WO-A1-2018/199196
- JP-A- 2016 110 296
- JP-A- 2016 184 296
- JP-A- 2016 184 296
- JP-A- 2016 224 086
- JP-A- 2017 161 645
- JP-B1- 6 425 287
- US-A1- 2019 247 719

## Description

This application is based upon and claims the benefit of priority from Japanese patent application No. 2019-000904, filed on January 7, 2019, and Japanese patent application No. 2019-085585, filed on April 26, 2019.

### TECHNICAL FIELD

The present invention relates to a rehabilitation support apparatus, a rehabilitation support system, a rehabilitation support method, and a rehabilitation support program.

### BACKGROUND ART

In the above technical field, patent literature 1 discloses a system configured to support rehabilitation performed for a hemiplegia patient of apoplexy or the like.

### CITATION LIST

### PATENT LITERATURE

Patent literature 1: Japanese Patent Laid-Open No. 2015-228957

### NON-PATENT LITERATURE

Non-patent literature 1: Nichols S, Patel H: Health and safety implications of virtual reality: a review of empirical evidence. Appl Ergon 2002; 33: 251-271

### FURTHER PATENT LITERATURE

WO 2018/199196 A1 discloses a system for actively updating an objective according to a rehabilitation action of a user, wherein a first rehabilitation action of the user is detected, and an avatar image that moves according to the detected first rehabilitation action and an objective image that indicates an objective of the first rehabilitation action are displayed. Next, the first rehabilitation action is compared with an objective position represented by the objective image to evaluate the rehabilitation ability of the user, and the objective position is updated according to the evaluation result. Further, a second rehabilitation action of the user who is performing the first rehabilitation action is detected, and the rehabilitation ability is evaluated on the basis of both the first rehabilitation action and the second rehabilitation action. If the evaluation of only the first rehabilitation action is at or above a certain level, the rehabilitation ability is evaluated on the basis of both the first rehabilitation action and the second rehabilitation action.
JP 2016 184296 A discloses that in a display control method, a computer executes processing of, when an image of a reference object is detected to be included in a first range of an acquired photographed image, displaying object data corresponding to the reference object, and when detection of inclusion of an image of the reference object in a second range of the photographed image has been continued for a predetermined period of time, even if inclusion of the image of the reference material in the first range of the photographed image is no longer detected, making a transition to a mode of continuing display of the object data corresponding to the reference material.
JP 2017 161645 A discloses a display control method including a computer executing a process to detect object data registered in correlation to a position inside an area specified in accordance with the position and direction of a head-mounted display detected by a sensor. The display control method includes a computer executing a process to display the object data on the head-mounted display when the object data is detected. The display control method also includes a computer executing a process to calculate the distance to a position correlated to each of a plurality of object data. The display control method also includes a computer executing a process to control the suppression of detecting the direction of the head-mounted display on the basis of the distribution of calculated distances.
JP 2016 110296 A discloses a wearable device which displays a display object indicating a relation positional relationship between a current location of a user and a position of an object, detects a physical condition of the user, and changes a visible degree of the display object on a display section, according to the detected physical condition.

### TECHNICAL PROBLEM

In the technique described in the above literature, however, the visual field of a user is narrowed by a display device attached to the head. More specifically, the visual field that was about 180° before the attachment of the display device is narrowed to about 110°.

In this case, a thing that was visible without turning the head before the attachment of the display device can be seen only when the head is turned. As a result, a difference is generated between visual information and inner ear information, which are input stimulations for the sense of equilibrium of a human, and the user may feel sick, like so-called car sickness. The phenomenon that a difference is generated between visual information and inner ear information by attaching a display device with a limited viewing angle is medically called sensory conflict.

For example, if a person encounters an earthquake without anything around him/her, nothing changes on the ground or background as "visual information", and information "not moving" is obtained as input from the visual sense. On the other hand, information of obviously sensing shakes is obtained as "inner ear information", and information "moving" is obtained as input from the inner ear. This is the mechanism that a person feels sick due to sensory conflict, and this phenomenon is known well as post earthquake dizziness syndrome (PEDS).

It is estimated that infants and children readily suffer motion sickness because even if "motion" is felt as the input from the inner ear, wrong information "not moving" is input from the visual sense because of the narrow visual field, and sensory conflict occurs. In this case, it is known well that motion sickness can be prevented by looking out of a window. This is a way of correctly obtaining input information from the visual sense. As a result, visual information and inner ear information are made to match, thereby preventing sensory conflict. In addition, for example, looking the landscape of the next moving destination from the head of a vehicle is also often used as a method of preventing motion sickness. This is a way of preventing sensory conflict by predicting, in advance based on visual information, a change in inner ear information that should be obtained next. Also, like motion sickness that a person can get used to, sensory conflict is considered to be a phenomenon that can be prevented by learning by causing a person to repetitively experience the same conditions.

That is, sensory conflict can be prevented by predicting (or learning), in advance, input information that should be obtained later.

There is recently a problem that when wearing a display device (HMD) with a limited visual field, a difference is generated between visual information and inner ear information, and the sense of equilibrium is distorted, resulting in sick feeling (VR motion sickness). It is known that since the visual field is limited, inputs different from visual information and inner ear information obtained in usual sense are performed, and sensory conflict occurs (non-patent literature 1).

The present invention enables to provide a technique of solving the above-described problem.

### SOLUTION TO PROBLEM

The invention is defined by the independent claims. Dependent claims specify embodiments of the invention.

The present invention provides a rehabilitation support apparatus according to claim 1.

The present invention also provides a rehabilitation support system according to claim 7.

Furthermore, the present invention provides a rehabilitation support method according to claim 8.

Moreover, the present invention provides a rehabilitation support program according to claim 9.

### ADVANTAGEOUS EFFECTS OF INVENTION

According to the present invention, it is possible to provide rehabilitation comfortable for a user.

### BRIEF DESCRIPTION OF DRAWINGS

Fig. 1 is a block diagram showing the arrangement of a rehabilitation support apparatus according to the first example embodiment;
Fig. 2 is a block diagram showing the arrangement of a rehabilitation support system according to the second example embodiment;
Fig. 3A is a view showing an example of the display screen of the rehabilitation support system according to the second example embodiment;
Fig. 3B is a view showing an example of the display screen of the rehabilitation support system according to the second example embodiment;
Fig. 3C is a view showing an example of the display screen of the rehabilitation support system according to the second example embodiment;
Fig. 3D is a view showing an example of the display screen of the rehabilitation support system according to the second example embodiment;
Fig. 3E is a view showing an example of the display screen of the rehabilitation support system according to the second example embodiment;
Fig. 3F is a view showing an example of the display screen of the rehabilitation support system according to the second example embodiment;
Fig. 3G is a view showing an example of the display screen of the rehabilitation support system according to the second example embodiment;
Fig. 4 is a view showing an example of the display screen of the rehabilitation support system according to the second example embodiment;
Fig. 5 is a flowchart showing the procedure of processing of the rehabilitation support system according to the second example embodiment;
Fig. 6 is a view showing another example of the display screen of the rehabilitation support system according to the second example embodiment;
Fig. 7 is a view showing an example of the operation screen of a rehabilitation support system according to the third example embodiment;
Fig. 8 is a view showing another example of the operation screen of the rehabilitation support system according to the third example embodiment; and
Fig. 9 is a view showing still other example of the operation screen of the rehabilitation support system according to the third example embodiment.

### DESCRIPTION OF EXAMPLE EMBODIMENTS

Example embodiments will now be described in detail with reference to the drawings. It should be noted that the relative arrangement of the components, the numerical expressions and numerical values set forth in these example embodiments do not limit the scope of the present invention unless it is specifically stated otherwise.

### [First Example Embodiment]

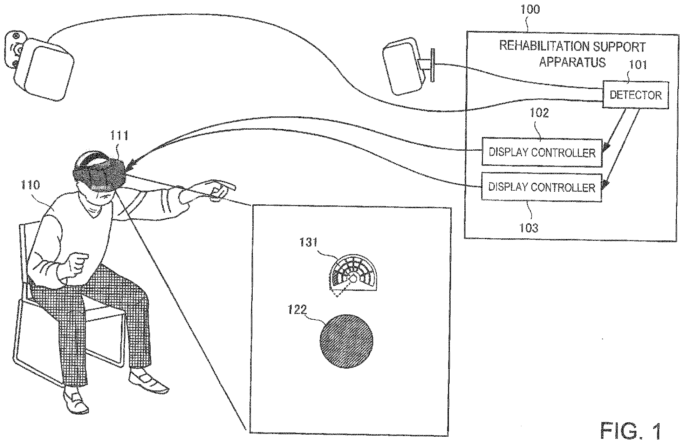

A rehabilitation support system 100 according to the first example embodiment will be described with reference to Fig. 1. The rehabilitation support system 100 is a system that supports rehabilitation performed for a user 110.

As shown in Fig. 1, the rehabilitation support system 100 includes a detector 101 and display controllers 102 and 103.

The detector 101 detects the direction of the head of the user 110 who wears a head mounted display 111.

The display controller 102 generates, in a virtual space, a rehabilitation target object 122 to be visually recognized by the user 110, and displays the target object 122 on the head mounted display 111 in accordance with the direction of the head of the user 110 detected by the detector 101. The display controller 103 displays, on the head mounted display 111, a notification image 131 used to notify the user of the position of the target object 122.

According to this example embodiment, the notification image is displayed, and the user is caused to predict, in advance based on visual information, a change in inner ear information that should be obtained next, thereby preventing sensory conflict.

### [Second Example Embodiment]

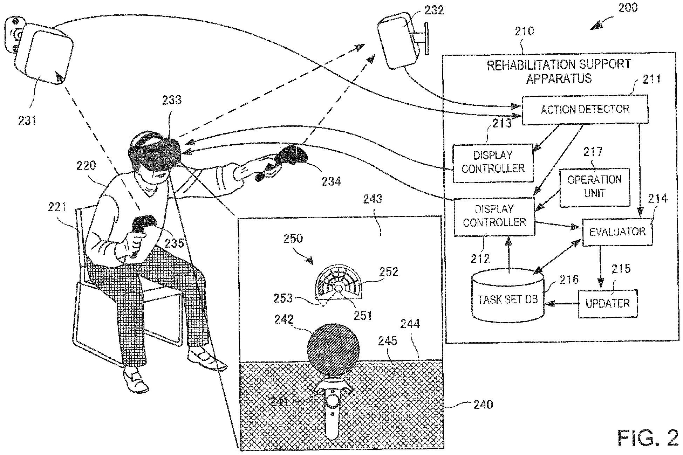

A rehabilitation supporting system 200 according to the second example embodiment will be described with reference to Fig. 2. Fig. 2 is a view for explaining the arrangement of a rehabilitation supporting system 200 according to this example embodiment.

As shown in Fig. 2, the rehabilitation supporting system 200 includes a rehabilitation support apparatus 210, two base stations 231 and 232, a head mounted display 233, and two controllers 234 and 235. A user 220 sitting on a chair 221 twists the upper half body or stretches the hands in accordance with display on the head mounted display 233, thereby making a rehabilitation action. In this example embodiment, a description will be made assuming rehabilitation performed while sitting on a chair. However, the present invention is not limited to this.

The two base stations 231 and 232 sense the motion of the head mounted display 233 and the motions of the controllers 234 and 235, and send these to the rehabilitation support apparatus 210. The rehabilitation support apparatus 210 performs display control of the head mounted display 233 while evaluating the rehabilitation action of the user 220.

Note that the head mounted display 233 can be of a non-transmissive type, a video see-through type, or optical see-through type.

**In** this example embodiment, as an example of a sensor configured to detect the position of the hand of the user, the controllers 234 and 235 held in the hands of the user 220, and the base stations 231 and 232 have been described. However, the present invention is not limited to this. A camera (including a depth sensor) configured to detect the positions of the hands of the user by image processing, a sensor configured to detect the positions of the hands of the user by a temperature, a wristwatch-type wearable terminal put on an arm of the user, a motion capture device, and the like are also included in the concept of the sensor according to the present invention.

The rehabilitation support apparatus 210 includes an action detector 211, display controllers 212 and 213, an evaluator 214, an updater 215, a task set database 216, and an operation unit 217.

The action detector 211 acquires, via the base stations 231 and 232, the positions of the controllers 234 and 235 held in the hands of the user 220, and detects the rehabilitation action of the user 220 based on changes in the positions of the hands of the user 220.

The display controller 212 generates, in a virtual space, an avatar object 241 that moves in accordance with a detected rehabilitation action and a target object 242 representing the target of the rehabilitation action. The display controller 212 displays, on a display screen 240, the images of the avatar object 241 and the target object 242 in accordance with the direction and position of the head mounted display 233 detected by the action detector 211. The images of the avatar object 241 and the target object 242 are superimposed on a background image 243. Here, the avatar object 241 has the same shape as the controllers 234 and 235. However, the shape is not limited to this. The avatar object 241 moves in the display screen 240 in accordance with the motions of the controllers 234 and 235. As displayed on the avatar object 241, buttons are prepared on the controllers 234 and 235, and the controllers 234 and 235 are configured to perform various kinds of setting operations. The background image 243 includes a horizon 244 and a ground surface image 245.

The display controller 212 displays the target object 242 while gradually changing the display position and size such that it falls downward from above the user 220. The user 220 moves the controllers 234 and 235, thereby making the avatar object 241 in the screen approach the target object 242. When a sensor object (not shown here) included in the avatar object 241 hits the target object 242, the target object 242 disappears.

Also, the display controller 213 displays a radar screen image 250 on the display screen 240 of the head mounted display 233. The radar screen image 250 shows the relative direction of the position of the target object 242 (here, initially set to the front direction of the user sitting straight on the chair) with respect to the reference direction in the virtual space.

The display controller 213 displays the radar screen image 250 at the center (for example, within the range of -50° to 50°) of the display screen 240 of the head mounted display 233 independently of the direction of the head of the user 220.

The radar screen image 250 includes a head image 251 representing the head of the user viewed from above, a block image 252 generated by dividing the periphery of the head image 251 into a plurality of blocks, and a fan-shaped image 253 as a visual field region image representing the visual field region of the user. A target position image representing the position of the target object is shown by coloring any block of the block image 252. This allows the user 220 to know whether the target object exists on the left side or right side with respect to the direction in which he/she faces. Note that in this example embodiment, the block image 252 is fixed, and the fan-shaped image 253 moves. However, the present invention is not limited to this, and the block image 252 may be moved in accordance with the direction of the head while fixing the fan-shaped image 253 and the head image 251. More specifically, if the head faces left, the block image 252 may rotate rightward.

The evaluator 214 compares the rehabilitation action detected by the action detector 211 and a target position represented by the target object displayed by the display controller 212, and evaluates the rehabilitation capability of the user 220. More specifically, it is decided, by comparing the positions in the three-dimensional virtual space, whether the target object 242 and the avatar object 241 that moves in correspondence with the rehabilitation action detected by the action detector 211 overlap.

If these overlap, it is evaluated that one rehabilitation action is cleared, and a point is added. The display controller 212 can make the target object 242 appear at different positions (for example, positions of three stages) in the depth direction. The evaluator 214 gives different points (a high point to a far object, and a low point to a close object).

The updater 215 updates a target task in accordance with the integrated point. For example, the target task may be updated using a task achieving ratio (target achieving count/task count).

The task set database 216 stores a plurality of task sets. A task represents one rehabilitation action that the user should make. More specifically, the task set database 216 stores, as information representing one task, information representing the size, position, and speed of a target object that was made to appear and the size of the avatar object at that time. The task set database 216 stores task sets each of which determines the order to provide the plurality of tasks to the user.

For example, the task sets may be stored as templates for each hospital, or a history of executed task sets may be stored for each user. The rehabilitation support apparatus 210 may be configured to be communicable with another rehabilitation support apparatus via the Internet. In this case, one task set may be executed by the same user in a plurality of places, or various templates may be shared by a plurality of users in remote sites.

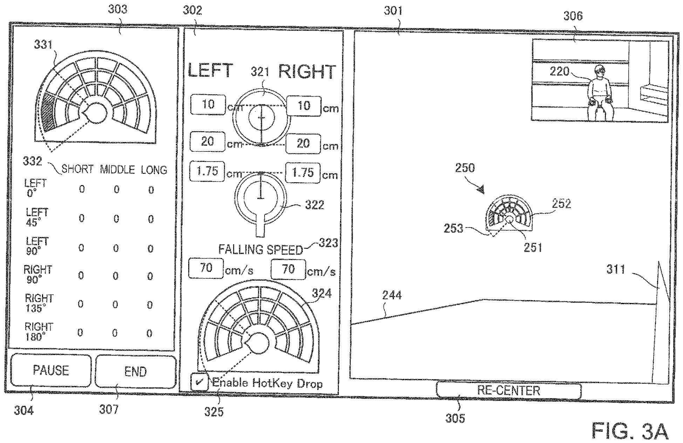

The operation unit 217 is provided to operate display control in the display controller 212 and the display controller 213. More specifically, the operation unit 217 displays an operation screen 300 for an operator, as shown in Figs. 3A to 3G. Here, the display that displays a setting screen can be any device, and may be an external display connected to the rehabilitation support apparatus 210 or a display incorporated in the rehabilitation support apparatus 210. The operation screen 300 includes a user visual field screen 301, a various parameter setting screen 302, a score display screen 303, a pause button 304, a re-center button 305, and an end button 307. For a descriptive convenience, Figs. 3A to 3G include a region 306 showing the actual state of the user 220. However, the operation screen 300 need not include the region 306.

The user visual field screen 301 shows an image actually displayed on the head mounted display 233. A reference direction 311 in the virtual space is displayed in the user visual field screen 301. As described with reference to Fig. 2, the radar screen image 250 is displayed at the center (for example, within the viewing angle range of -50° to 50°) of the user visual field screen 301. The radar screen image 250 shows the relative direction of the position of the target object 242 that appears next with respect to the reference direction in the virtual space. In this example, the coloring position in the block image 252 represents that the target object 242 appears at the farthest position on the left side with respect to the reference direction 311 in the virtual space. Based on the position of the fan-shaped image 253 and the direction of the head image 251, it can be seen that the user already faces left.

The various parameter setting screen 302 is a screen configured to set a plurality of parameters for defining a task. The operation unit 217 accepts input to the various parameter setting screen 302 from an input device (not shown). The input device may be a mouse or a keyboard, or may be a touch panel, and can use any technical component.

The various parameter setting screen 302 includes an input region 321 that decides the sizes of left and right target objects, an input region 322 that decides the size of the avatar object 241, an input region 323 that decides the falling speed of the target object, and an input region 324 that decides the position of a target object that appears next. The various parameter setting screen 302 also includes a check box 325 that sets whether to accept an operation of a target object appearance position by a hot key.

The input region 321 can set, on each of the right and left sides, the radius (visual recognition size) of a visual recognition object that makes the target object position easy for the user to see, and the radius (evaluation size) of a target object that reacts with the avatar object 241. That is, in the example shown in Fig. 3A, the user can see a ball with a radius of 20 cm. Actually, the task is completed only when he/she has touched a ball with a radius of 10 cm located at the center of the ball. If the visual recognition size is small, it is difficult for the user to find the target object. If the visual recognition size is large, the user can easily find the target object. If the evaluation size is large, the allowable amount of the deviation of the avatar object 241 is large. If the evaluation size is small, the allowable amount of the deviation of the avatar object 241 is small, and a rehabilitation action can be evaluated more severely. The visual recognition sizes and the evaluation sizes may be made to match.

In the input region 322, the sensor size of the avatar object 241 (the size of the sensor object) can separately be set on the left and right sides. If the sensor size is large, a task is achieved even if the position of a hand largely deviates from the target object. Hence, the difficulty of the rehabilitation action is low. Conversely, if the sensor size is small, it is necessary to correctly move the hand to the center region of the target object (depending on the evaluation size). Hence, the difficulty of the rehabilitation action is high. In the example shown in Fig. 3A, the sensor sizes are 1.75 cm on the left and right sides.

In the input region 323, the speed of the target object 242 moving in the virtual space can be defined on each of the left and right sides. In this example the speed is set to 70 cm/s.

That is, in the example shown in Fig. 3A, the task of the user 220 is grabbing an avatar object (controller) including a sensor portion with a size of 1.75 cm in the virtual space and making it contact a target object (ball) that has a radius of 10 cm and falls at a speed of 70 cm/s in a far place on the left side.

The input region 324 has the shape of the enlarged radar screen image 250. Since the check box 325 has a check mark, if an operation of clicking or tapping one of a plurality of blocks in the input region 324 is performed, the target object 242 is generated at a position in the virtual space corresponding to the position of the block for which the operation is performed.

On the other hand, the score display screen 303 includes an enlarged radar screen image 331 and a score list 332. The enlarged radar screen image 331 is obtained by enlarging the radar screen image 250 displayed on the head mounted display 233, and changes in real time in accordance with the motion of the user and the position where the target object 242 appears next.

The score list 332 shows the total number of tasks at each position, and points representing how many times each task has been achieved. The point may be expressed as a fraction, a percentage, or a combination thereof. After a series of rehabilitation actions defined by one task set, the evaluator 214 derives a rehabilitation evaluation point using the values in the score list 332.

At this time, the evaluator 214 adds a weight to a point based on the target object appearance position, the accuracy of touch by the avatar object 241, and the continues achievement count. In this example embodiment, the falling speed and the task interval are not included in the materials of weighting. This is based on a fact that the exercise intensity does not always depend on the speed, and also, factors that make the user hurry are excluded from the viewpoint of preventing accidents.

The pause button 304 is a button used to pause a series of rehabilitation actions. The end button 307 is a button used to end a series of rehabilitation actions.

The re-center button 305 is a button that accepts, from the operator, a reconstruction instruction to reconstruct the virtual space in accordance with the position of the user 220. When the re-center button 305 is operated, the display controller 212 reconstructs the virtual space in which the position of the head mounted display 233 at the instant is set to the origin, and the direction of the head mounted display 233 at the instant is set to the reference direction.

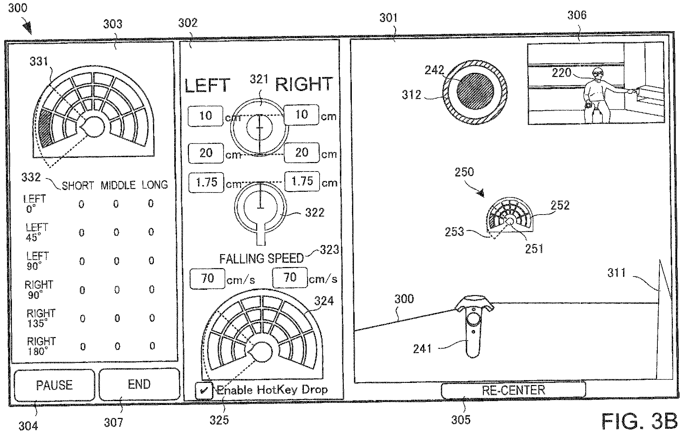

Fig. 3B shows a state in which the target object 242 appears in the user visual field screen 301 shown in Fig. 3A. In this state, when the user 220 stretches the left arm, as shown in the region 306, the avatar object 241 appears in the user visual field screen 301. A visual recognition object 312 that raises the visibility of the target object 242 is displayed around the target object 242. Here, the visual recognition object 312 has a doughnut shape. However, the present invention is not limited to this, and a line or arrow that radially extends from the target object may be used. The visual recognition size set in the input region 321 is the radius of the visual recognition object 312 with the doughnut shape.

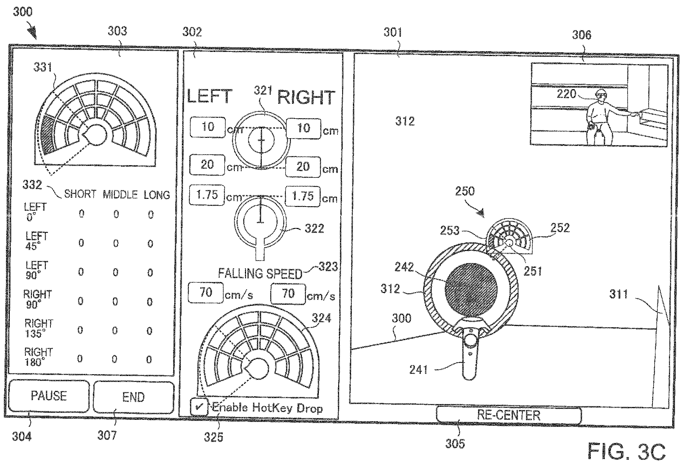

Fig. 3C shows the user visual field screen 301 at the instant when the target object 242 shown in Fig. 3B further falls and contacts the avatar object 241. At this point of time, the avatar object 241 and the visual recognition object 312 are in contact. At this point of time, however, the target object 242 does not disappear, and the task is not achieved (a predetermined point is given, and good evaluation is obtained). Only when the avatar object 241 contacts the target object 242, the task is achieved (perfect evaluation).

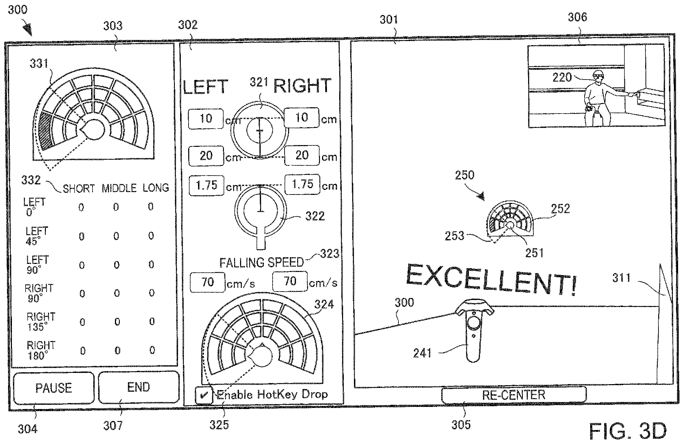

Fig. 3D shows the user visual field screen 301 immediately after the target object 242 in Fig. 3C further falls, contacts the avatar object 241, and disappears.

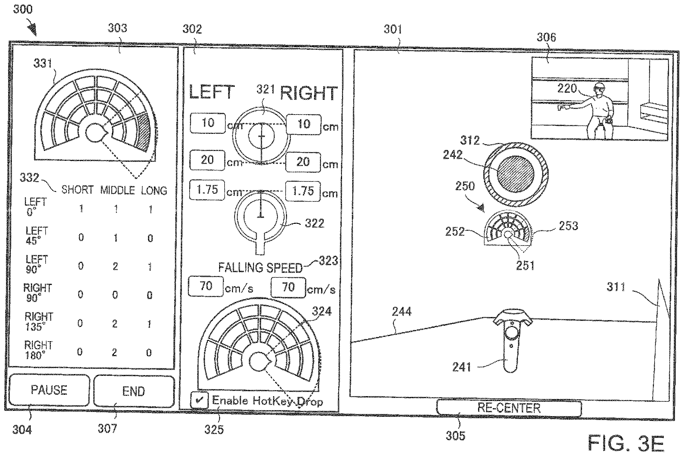

Fig. 3E shows the operation screen 300 in which the user has a task of grabbing an avatar object (controller) including a sensor portion with a size of 1.75 cm in the virtual space and making it contact a target object (ball) that has a radius of 10 cm and falls at a speed of 70 cm/s in a far place on the right side when viewed from the user.

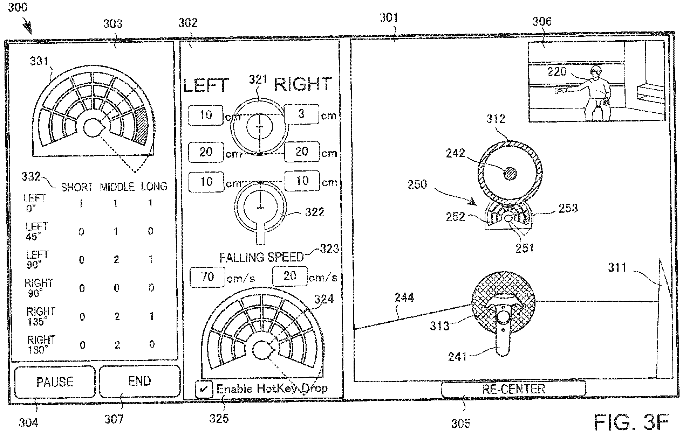

Fig. 3F shows the operation screen 300 in which the size of the target object on the right side is changed to 3 cm, and the sensor size is changed to 10 cm. Since the sensor size is as large as 10 cm, a sensor object 313 protrudes from the avatar object 241 and can visually be recognized. The user has a task of grabbing an avatar object (controller) including the sensor object 313 with a size of 10 cm in the virtual space and making it contact a target object (ball) that has a radius of 3 cm and falls at a speed of 20 cm/s in a far place on the right side when viewed from the user.

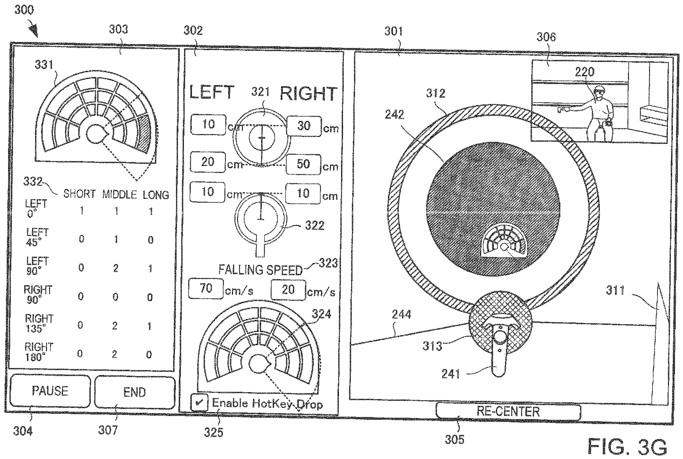

Fig. 3G shows the operation screen 300 in which the size of the target object on the right side is changed to 30 cm, and the sensor size is changed to 10 cm. The user has a task of grabbing an avatar object (controller) including the sensor object 313 with a size of 10 cm in the virtual space and making it contact a target object (ball) that has a radius of 30 cm and falls at a speed of 20 cm/s in a far place on the right side when viewed from the user.

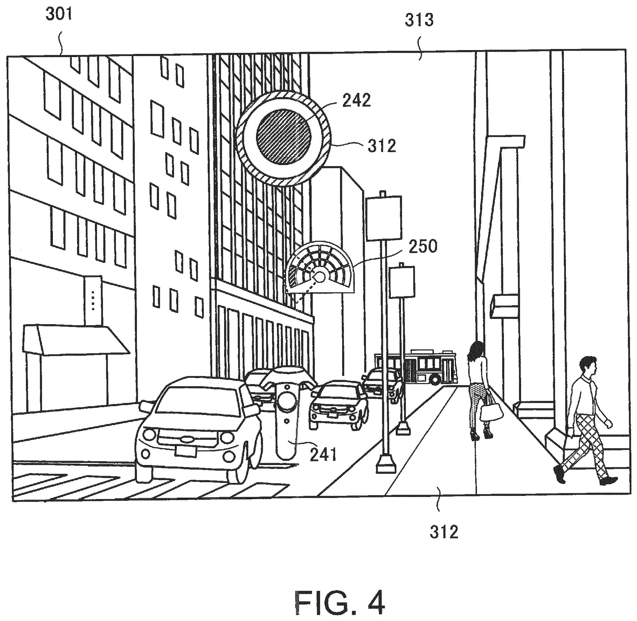

As shown in Fig. 4, a landscape image (for example, a moving image of a street in New York) obtained by capturing an actual landscape may be displayed as the background image. As the landscape image, a video of a road around a rehabilitation facility may be used, or a video of an abroad may be used. This causes the user to feel like he/she has a walk in a foreign land or feel like he/she has a walk in a familiar place. When the landscape image is superimposed, it is possible to implement a training in a situation with an enormous amount of information while entertaining a patient.

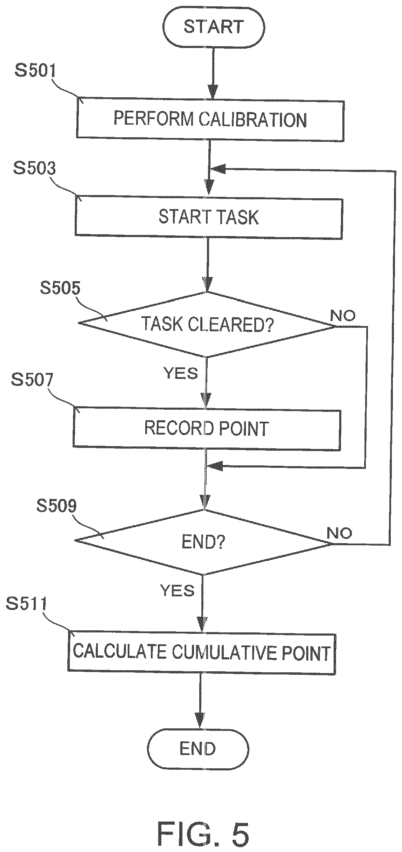

Fig. 5 is a flowchart showing the procedure of processing of the rehabilitation support apparatus 210.

In step S501, as calibration processing, the target of a rehabilitation action is initialized in accordance with the user. More specifically, each patient is first made to do a work within an action enable range as calibration. It is set to the initial value, and the target is initialized in accordance with the user.

Next, in step S503, a task is started. More specifically, in addition to a method of designating each task in real time by an operator, a task set called a template may be read out from the task set database, and a task request (that is, display of a target object by the display controller 212) may be issued sequentially for a plurality of preset tasks.

In step S505, it is determined whether the task is cleared. If the task is cleared, a point is recorded in accordance with the degree of clear (perfect or good).

In step S507, it is determined whether all tasks of a task set are ended, or the operator presses the end button 307.

If all the tasks are not ended, the process returns to step S503 to start the next task. If all the tasks are ended, the process advances to step S511 to add a weight by conditions to the point of each task and calculate a cumulative point.

In step S507, the degree of fatigue of the user is calculated based on a change in the point and the like. If the degree of fatigue exceeds a predetermined threshold, the processing may automatically be ended based on the "stop condition".

### (Dual Task)

A healthy person simultaneously makes two or more actions such as "walk while talk" in a daily life. The "capability of simultaneously making two actions" declines with age. For example, "stop when talked during walking" or the like occurs. It is considered that not only "deterioration of the motor function" but also the "decline of capability of simultaneously making two actions" are related to the cause of fall of an aged person. In fact, even if it is judged that the motor function has sufficiently recovered by rehabilitation, many aged persons fall after coming home. One of the reasons is that rehabilitation is performed in a state in which the environment/condition for enabling concentration to rehabilitation actions is prepared. That is, in a living environment, factors that impede concentration to actions exist, and, for example, many actions are made under a condition that visibility is poor, an obstacle exists, or attention is paid to conversation.

It is therefore considered that it is important to perform rehabilitation that distracts attention and, more specifically, a dual task is preferably given when performing a training. The dual task training is a program effective to prevent not only fall of an aged person but also dementia.

The dual task training includes not only a training that combines a cognitive task and a motion task but also a training that combines two motion tasks.

The task described above with reference to Figs. 3A to 3G is a dual task of cognition + motion because it is necessary to confirm the position of the next target object on the radar screen image (cognition task) and direct the body to the position and stretch the hand at an appropriate timing (motion task).

As another cognition task + motion task, a training of stretching the hand while sequentially subtracting 1 from 100 or a training of stretching the hand while avoiding spilling of water from a glass is possible.

If the evaluation lowers by about 20% in a dual task motion inspection as compared to a simple motion, the evaluator 214 notifies the display controller 212 to repeat the dual task.

### (Other Dual Task Training Examples)

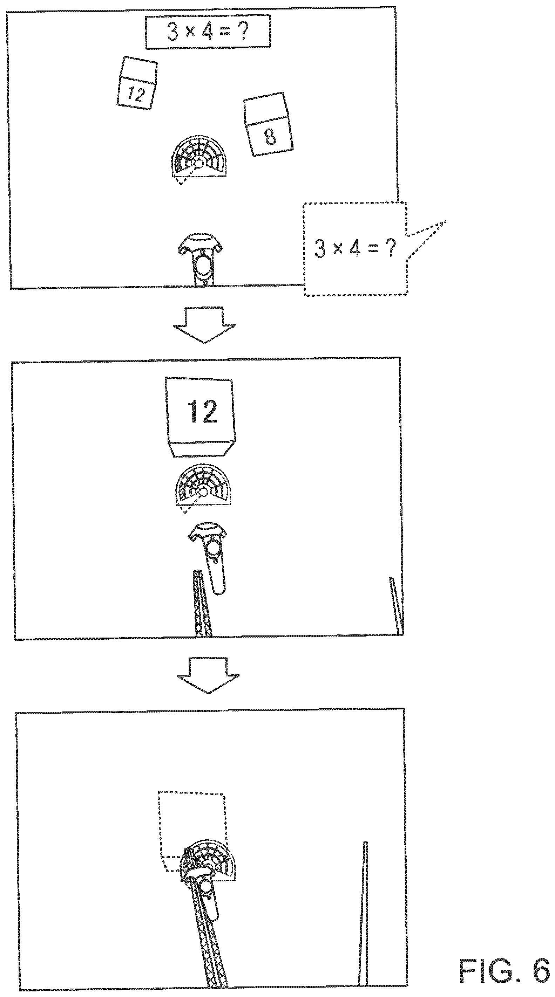

As shown in Fig. 6, a question image (for example, multiplication) may be displayed on the background screen in a superimposed manner, or a question may be asked by voice, and in a plurality of target objects, acquisition of a target object with an answer displayed thereon may be evaluated high. One of rock, scissors, and paper may be displayed on the background screen, and collection of an object with a mark to win displayed thereon may be requested. Such a choice type task + motion task is a dual task training that requests a more advanced cognitive function.

Alternatively, a number may simply be displayed on an object, and only acquisition of an object of a large number may be evaluated.

The evaluator 214 may compare the point of a single task and the point of a dual task and evaluate the cognitive function using the point difference.

As a dual task simultaneously requesting two motor functions, for example, two foot pedals may be prepared, and collection of a target object may be possible only when one of the foot pedals is stepped. For a target object coming from the right side, the user needs to stretch the right hand while stepping the foot pedal with the right foot. Hence, double motor functions of synchronizing the hand and foot are required. In addition, the user may be requested to make an avatar object on the opposite side of an avatar object on the object collecting side always touch a designated place.

As described above, according to this example embodiment, since the direction to which the user should direct the head can be recognized in advance by the radar screen image, even if the visual field is narrow, no confusion occurs in inner ear information, and VR motion sickness can be prevented. That is, it is possible to provide rehabilitation comfortable for the user during rehabilitation using a head mounted display.

Note that the position of the next object may be notified using a stereoscopic sound.

Note that the target object need not always vertically fall, and the user may touch a target that horizontally moves to the near side.

### [Third Example Embodiment]

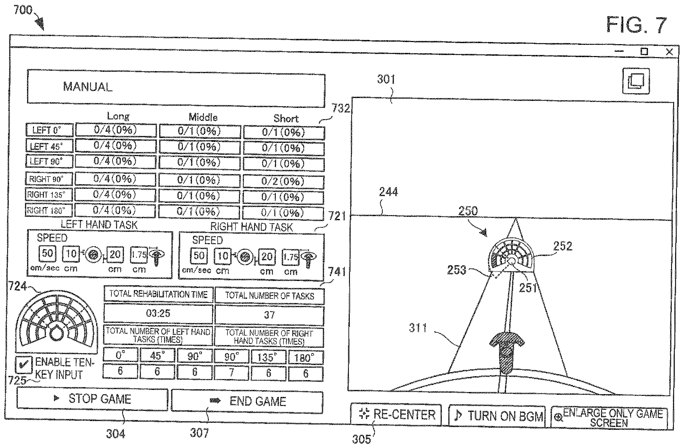

A rehabilitation support apparatus according to the third example embodiment will be described next with reference to Figs. 7 to 9. Figs. 7 to 9 are views showing operation screens 700, 800, and 900 of the
rehabilitation support apparatus. The rehabilitation support apparatus according to this example embodiment is different from the second example embodiment in the layout of the operation screen. The rest of the components and operations is the same as in the second example embodiment. Hence, the same reference numerals denote the same components and operations, and a detailed description thereof will be omitted.

Fig. 7 is a view showing the operation screen 700 configured to generate a task and provide it to a user based on the manual operation of an operator. A user visual field screen 301, a re-center button 305, and the like and the contents thereof are the same as in the screen described with reference to Fig. 3A.

The operation screen 700 includes, on the left side, a score display region 732 that displays the positions of target objects that were made to appear in the past and their achieving counts (achieving ratios), a various parameter setting region 721, and a task history display region 741. The operation screen 700 also includes an input region 724 configured to decide the position of a target object that appears next, and a check box 725 that sets whether to accept an operation of a target object appearance position by a hot key.

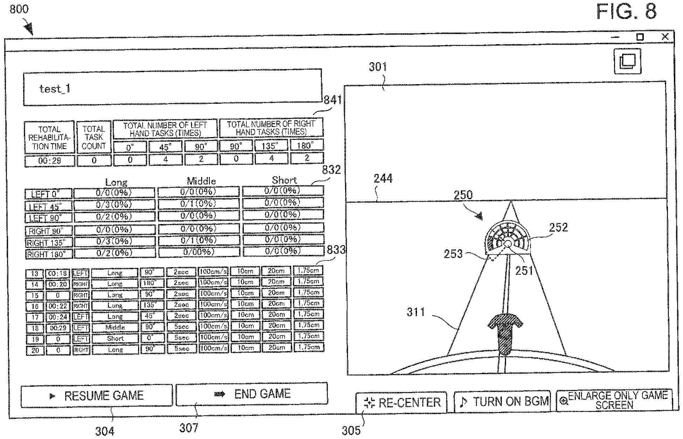

Fig. 8 is a view showing the operation screen 800 in a case in which the user is caused to execute a task set read out from a task set database 216. The user visual field screen 301, the re-center button 305, and the like and the contents thereof are the same as in the screen described with reference to Fig. 3A.

The operation screen 800 does not include a various parameter setting region, and includes a task history display region 841, a score display region 832 that displays the positions of target objects that were made to appear in the past and their achieving counts (achieving ratios), and a task list display region 833 of task sets to be generated later.

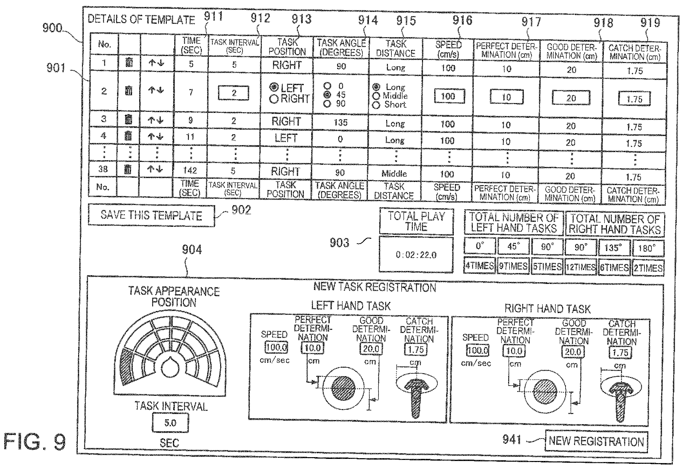

Fig. 9 is a view showing display of the contents of task sets stored in the task set database 216 and an example of the editing screen 900. On the screen shown in Fig. 9, a task set 901 formed by 38 tasks is displayed.

Each task included in the task set 901 has, as parameters, a generation time 911, a task interval 912, a task position 913, a task angle 914, a task distance 915, a speed 916, a perfect determination criterion 917, a good determination criterion 918, and a catch determination criterion 919.

Fig. 9 shows a state in which the parameters of task No. 2 are to be edited. When a save button 902 is selected after editing, the task set in the task set database 216 is updated.

In a totalization data display region 903, the total play time and the total numbers of tasks of the left and right hands are displayed.

In a new task registration region 904, a new task in which various kinds of parameters are set can be added to the task set 901. When all parameters such as the appearance position and speed are set, and a new registration button 941 is selected, one row is added to the task set 901 on the upper side. Here, the task is registered as task No. 39.

As described above, in this example embodiment, it is possible to provide a user-friendly rehabilitation support apparatus with higher operability.

### [Other Example Embodiments]

While the invention has been particularly shown and described with reference to example embodiments, the invention is not limited to these example embodiments. It will be understood by those of ordinary skill in the art that various changes in form and details may be made therein without departing from the scope of the present invention as defined by the claims.

The present invention is applicable to a system including a plurality of devices or a single apparatus. The present invention is also applicable even when a rehabilitation support program for implementing the functions of example embodiments is supplied to the system or apparatus directly or from a remote site. Hence, the present invention also incorporates the program installed in a computer to implement the functions of the present invention by the computer, a medium storing the program, and a WWW (World Wide Web) server that causes a user to download the program. Especially, the present invention incorporates at least a non-transitory computer readable medium storing a program that causes a computer to execute processing steps included in the above-described example embodiments.

## Claims

1. A rehabilitation support apparatus (210) comprising:
a detector (101, 211) that is configured to detect a direction of a head of a user (110, 220) wearing a head mounted display (111, 233);
a first display controller (102, 212) that is configured to generate, in a virtual space, a rehabilitation target object (122, 242) to be visually recognized by the user (110, 220) and cause display of the rehabilitation target object on a display screen (340) of the head mounted display (111, 233) in accordance with the direction of the head of the user detected by the detector, wherein the first display controller (102, 212) displays the rehabilitation target object (122, 242) while gradually changing a display position and size of the rehabilitation target object (122, 242) such that it falls downward from above the user (220); and
a second display controller (103, 213) that is configured to cause display, on the display screen (340) of the head mounted display (111, 233), of a notification image (131) used to notify the user (110, 220) of the display position of the rehabilitation target object;
wherein the second display controller (103, 213) is configured to cause display of a reference direction (311) in the virtual space on the display screen (340) of the head mounted display (111,233), wherein the reference direction indicates the direction of the head of the user (110, 220) wearing the head mounted display (111, 233) when a re-center button is operated by an operator;
wherein the notification image (131) is a radar screen image (250) that is configured to show a relative direction of the position of the rehabilitation target object with respect to the reference direction in the virtual space; wherein the notification image (131) is the radar screen image that is configured to show a relative direction of the position of the rehabilitation target object with respect to the direction of the head of the user (110, 220); and
wherein the radar screen image (250) includes a visual field region image (253) representing a visual field of the user (110, 220), and a target position image representing the position of the rehabilitation target object.

2. The rehabilitation support apparatus (210) according to claim 1, wherein the second display controller (103, 213) is configured to cause display the notification image (131) at a center portion of the display screen of the head mounted display (111, 233) independently of the direction of the head of the user (110, 220).

3. The rehabilitation support apparatus (210) according to any one of claims 1 to 2, wherein the notification image (131) includes a head image representing the direction of the head of the user (110, 220) viewed from above, and the target position image representing the position of the rehabilitation target object.

4. The rehabilitation support apparatus (210) according to any one of claims 1 to 3, wherein the notification image (131) includes a block image generated by dividing a periphery of the user (110, 220) into a plurality of blocks.

5. The rehabilitation support apparatus (210) according to any one of claims 1 to 4, further comprising an operation unit (217) used by the operator to operate display control by the first display controller (102, 212) and the second display controller (103, 213),
wherein the operation unit (217) is configured to display, for the operator, an operation screen (300).

6. The rehabilitation support apparatus (210) according to claim 5, wherein the operation unit is configured to accept from the re-center button (305), which is a button that accepts, from the operator, a reconstruction instruction to reconstruct the virtual space in accordance with the position of the user (110, 220), and in accordance with the reconstruction instruction, the first display controller (102, 212) reconstructs the virtual space in which the position of the head mounted display (111, 233) at the instant is set to the origin, and the direction of the head mounted display (111, 233) at the instant is set to the reference direction.

7. A rehabilitation support system (200) comprising:
a rehabilitation support apparatus (210) according to any one of claims 1 to 6;
a head mounted display (111, 233);
a sensor that is configured to detect a position of a hand of a user (110, 220); and,
an evaluator (214) that is configured to compare the position of a hand of the user (110, 220) captured by the sensor with a target position represented by the rehabilitation target object and evaluates a capability of the user (110, 220).

8. A rehabilitation support method performed by a rehabilitation support apparatus (210) comprising a detector (101, 211), a first display controller (102, 212), and a second display controller (103, 213), the rehabilitation support method comprising:
Detecting, by the detector (101, 211), a direction of a head of a user (110, 220) wearing a head mounted display (111, 233);
generating, by the first display controller (102, 212), in a virtual space, a rehabilitation target object (122, 242) to be visually recognized by the user (110, 220) and displaying the rehabilitation target object on a display screen (340) of the head mounted display (111, 233) in accordance with the direction of the head of the user detected in the detecting, wherein the first display controller (102, 212) displays the rehabilitation target object (122,242), while gradually changing a display position and size of the rehabilitation target object (122, 242) such that it falls downward from above the user (220); and
displaying, by the second display controller (103, 213), on the display screen (340) of the head mounted display (111, 233), a notification image (131) used to notify the user (110, 220) of the display position of the rehabilitation target object;
wherein the second display controller (103, 213) is configured to cause display of a reference direction (311) in the virtual space on the display screen (340) of the head mounted display (111,233), wherein the reference direction indicates the direction of the head of the user (110, 220) wearing the head mounted display (111, 233) when a re-center button is operated by an operator;
wherein the notification image (131) is a radar screen image (250) that is configured to show a relative direction of the position of the rehabilitation target object with respect to a reference direction in the virtual space;
wherein the notification image (131) is the radar screen image that is configured to show a relative direction of the position of the rehabilitation target object with respect to the direction of the head of the user (110, 220); and
wherein the radar screen image (250) includes a visual field region image (253) representing a visual field of the user (110, 220), and a target position image representing the position of the rehabilitation target object.

9. A rehabilitation support program comprising a plurality of computer readable instructions that when executed by a computer causes the computer to execute the steps of the method of claim 8.

## Patentansprüche

1. Rehabilitationsunterstützungsgerät (210), umfassend:
einen Detektor (101, 211), der dazu konfiguriert ist, eine Richtung eines Kopfs eines Benutzers (110, 220) zu detektieren, der ein Head-Mounted-Display (111, 233) trägt;
einen ersten Displaycontroller (102, 212), der dazu konfiguriert ist, in einem virtuellen Raum ein Rehabilitationszielobjekt (122, 242) zu generieren, das durch den Benutzer (110, 220) visuell zu erkennen ist, und eine Anzeige des Rehabilitationszielobjekts auf einem Displayschirm (340) des Head-Mounted-Display (111, 233) in Übereinstimmung mit der Richtung des Kopfs des Benutzers zu bewirken, die durch den Detektor detektiert ist, wobei der erste Displaycontroller (102, 212) das Rehabilitationszielobjekt (122, 242) während einer schrittweisen Veränderung einer Anzeigeposition und Größe des Rehabilitationszielobjekts (122, 242) derart anzeigt, das es von oberhalb des Benutzers (220) nach unten fällt; und
einen zweiten Displaycontroller (103, 213), der dazu konfiguriert ist, eine Anzeige, auf dem Displayschirm (340) des Head-Mounted-Display (111, 233), eines Benachrichtigungsbilds (131) zu bewirken, das dazu verwendet wird, den Benutzer (110, 220) über die Anzeigeposition des Rehabilitationszielobjekts zu benachrichtigen;
wobei der zweite Displaycontroller (103, 213) dazu konfiguriert ist, eine Anzeige einer Referenzrichtung (311) in dem virtuellen Raum auf dem Displayschirm (340) des Head-Mounted-Display (111, 233) zu bewirken, wobei die Referenzrichtung die Richtung des Kopfs des Benutzers (110, 220) angibt, der das Head-Mounted-Display (111, 233) trägt, wenn ein Rezentrierungsknopf durch einen Bediener betätigt wird;
wobei das Benachrichtigungsbild (131) ein Radarschirmbild (250) ist, das dazu konfiguriert ist, eine relative Richtung der Position des Rehabilitationszielobjekts mit Bezug zu der Referenzrichtung in dem virtuellen Raum zu zeigen; wobei das Benachrichtigungsbild (131) das Radarschirmbild ist, das dazu konfiguriert ist, eine relative Richtung der Position des Rehabilitationszielobjekts mit Bezug zu der Richtung des Kopfs des Benutzers (110, 220) zu zeigen; und
wobei das Radarschirmbild (250) ein Sichtfeldregionbild (253) enthält, das ein Sichtfeld des Benutzers (110, 220) repräsentiert, und ein Zielpositionsbild, das die Position des Rehabilitationszielobjekts repräsentiert.

2. Rehabilitationsunterstützungsgerät (210) nach Anspruch 1, wobei der zweite Displaycontroller (103, 213) dazu konfiguriert ist, eine Anzeige des Benachrichtigungsbilds (131) in einem Zentralbereich des Displayschirms des Head-Mounted-Display (111, 233) unabhängig von der Richtung des Kopfs des Benutzers (110, 220) zu bewirken.

3. Rehabilitationsunterstützungsgerät (210) nach einem der Ansprüche 1 bis 2, wobei das Benachrichtigungsbild (131) ein Kopfbild enthält, das die Richtung des Kopfs des Benutzers (110, 220) von oben betrachtet repräsentiert, und das Zielpositionsbild, das die Position des Rehabilitationszielobjekts repräsentiert.

4. Rehabilitationsunterstützungsgerät (210) nach einem der Ansprüche 1 bis 3, wobei das Benachrichtigungsbild (131) ein Blockbild enthält, das durch Unterteilen einer Peripherie des Benutzers (110,220) in eine Mehrzahl von Blöcken generiert ist.

5. Rehabilitationsunterstützungsgerät (210) nach einem der Ansprüche 1 bis 4, ferner umfassend eine Betätigungseinheit (217), die durch den Bediener verwendet wird, um eine Displaysteuerung durch den ersten Displaycontroller (102, 212) und den zweiten Displaycontroller (103, 213) zu betätigen, wobei die Bedieneinheit (217) dazu konfiguriert ist, für den Bediener einen Bedienungsschirm (300) anzuzeigen.

6. Rehabilitationsunterstützungsgerät (210) nach Anspruch 5, wobei die Bedieneinheit für eine Annahme von dem Rezentrierungsknopf (305) konfiguriert ist, der ein Knopf ist, der von dem Bediener eine Rekonstruktionsanweisung zum Rekonstruieren des virtuellen Raums in Übereinstimmung mit der Position des Benutzers (110, 220) annimmt, und wobei in Übereinstimmung mit der Rekonstruktionsanweisung der erste Displaycontroller (102, 212) den virtuellen Raum rekonstruiert, in dem die Position des Head-Mounted-Display (111, 233) zu dem Zeitpunkt auf den Ursprung gesetzt ist, und die Richtung des Head-Mounted-Display (111, 233) zu dem Zeitpunkt auf die Referenzrichtung gesetzt ist.

7. Rehabilitationsunterstützungssystem (200), umfassend:
ein Rehabilitationsunterstützungsgerät (210) nach einem der Ansprüche 1 bis 6;
ein Head-Mounted-Display (111, 233);
einen Sensor, der zum Detektieren einer Position einer Hand eines Benutzers (110, 220) konfiguriert ist; und
eine Evaluierungseinrichtung (214), die dazu konfiguriert ist, die Position einer Hand des Benutzers (110, 220), die durch den Sensor erfasst ist, mit einer Zielposition zu vergleichen, die durch das Rehabilitationszielobjekt repräsentiert ist, und eine Fähigkeit des Benutzers (110, 220) evaluiert.

8. Rehabilitationsunterstützungsverfahren, das durch ein Rehabilitationsunterstützungsgerät (210) durchgeführt wird, umfassend einen Detektor (101, 211), einen ersten Displaycontroller (102, 212), und einen zweiten Displaycontroller (103, 213), wobei das Rehabilitationsunterstützungsverfahren umfasst:
Detektieren, durch den Detektor (101, 211), einer Richtung eines Kopfs eines Benutzers (110, 220), der ein Head-Mounted-Display (111, 233) trägt;
Generieren, durch den ersten Displaycontroller (102, 212) und in einem virtuellen Raum, eines Rehabilitationszielobjekts (122, 242), das durch den Benutzer (110, 220) visuell zu erkennen ist, und Anzeigen des Rehabilitationszielobjekts auf einem Displayschirm (340) des Head-Mounted-Display (111, 233) in Übereinstimmung mit der Richtung des Kopfs des Benutzers, die beim Detektieren detektiert wird, wobei der erste Displaycontroller (102, 212) das Rehabilitationszielobjekt (122, 242) während einer schrittweisen Veränderung einer Anzeigeposition und Größe des Rehabilitationszielobjekts (122, 242) derart anzeigt, das es von oberhalb des Benutzers (220) nach unten fällt; und
Anzeigen durch den zweiten Displaycontroller (103, 213) und auf dem Displayschirm (340) des Head-Mounted-Display (111, 233), eines Benachrichtigungsbilds (131), das dazu verwendet wird, den Benutzer (110, 220) über die Anzeigeposition des Rehabilitationszielobjekts zu benachrichtigen;
wobei der zweite Displaycontroller (103, 213) dazu konfiguriert ist, eine Anzeige einer Referenzrichtung (311) in dem virtuellen Raum auf dem Displayschirm (340) des Head-Mounted-Display (111, 233) zu bewirken, wobei die Referenzrichtung die Richtung des Kopfs des Benutzers (110, 220) angibt, der das Head-Mounted-Display (111, 233) trägt, wenn ein Rezentrierungsknopf durch einen Bediener betätigt wird;
wobei das Benachrichtigungsbild (131) ein Radarschirmbild (250) ist, das dazu konfiguriert ist, eine relative Richtung der Position des Rehabilitationszielobjekts mit Bezug zu der Referenzrichtung in dem virtuellen Raum zu zeigen;
wobei das Benachrichtigungsbild (131) das Radarschirmbild ist, das dazu konfiguriert ist, eine relative Richtung der Position des Rehabilitationszielobjekts mit Bezug zu der Richtung des Kopfs des Benutzers (110, 220) zu zeigen; und
wobei das Radarschirmbild (250) ein Sichtfeldregionbild (253) enthält, das ein Sichtfeld des Benutzers (110, 220) repräsentiert; und ein Zielpositionsbild, das die Position des Rehabilitationszielobjekts repräsentiert.

9. Rehabilitationsunterstützungsprogramm, das eine Mehrzahl von computerlesbaren Anweisungen umfasst, die bei Ausführung durch einen Computer den Computer dazu veranlassen, die Schritte des Verfahrens von Anspruch 8 auszuführen.

## Revendications

1. Un appareil (210) d'aide à la rééducation, comprenant :
un détecteur (101, 211), qui est configuré pour détecter la direction de la tête d'un utilisateur (110, 220) portant un casque de réalité virtuelle (111, 233) ;
un premier contrôleur d'affichage (102, 212), qui est configuré pour générer, dans un espace virtuel, un objet cible de rééducation (122, 242) destiné à être reconnu visuellement par l'utilisateur (110, 220) et pour provoquer l'affichage de l'objet cible de rééducation sur un écran d'affichage (340) du casque de réalité virtuelle (111, 233) conformément à la direction de la tête de l'utilisateur détectée par le détecteur, le premier contrôleur d'affichage (102, 212) affichant l'objet cible de rééducation (122, 242) tout en modifiant progressivement la position et la taille d'affichage de l'objet cible de rééducation (122, 242) de telle sorte qu'il tombe vers le bas depuis au-dessus de l'utilisateur (220) ; et
un deuxième contrôleur d'affichage (103, 213), qui est configuré pour provoquer l'affichage, sur l'écran d'affichage (340) du casque de réalité virtuelle (111, 233), d'une image de notification (131) utilisée pour notifier à l'utilisateur (110, 220) la position d'affichage de l'objet cible de rééducation ;
le deuxième contrôleur d'affichage (103, 213) est configuré pour provoquer l'affichage d'une direction de référence (311) dans l'espace virtuel sur l'écran d'affichage (340) du casque de réalité virtuelle (111, 233), la direction de référence indiquant la direction de la tête de l'utilisateur (110, 220) portant le casque de réalité virtuelle (111, 233) lorsqu'un bouton de recentrage est actionné par un opérateur ;
l'image de notification (131) est une image d'écran radar (250) qui est configurée pour montrer une direction relative de la position de l'objet cible de rééducation par rapport à la direction de référence dans l'espace virtuel ; l'image de notification (131) est l'image d'écran radar qui est configurée pour montrer une direction relative de la position de l'objet cible de rééducation par rapport à la direction de la tête de l'utilisateur (110, 220) ; et
l'image d'écran radar (250) comprend une image de zone de champ visuel (253) représentant un champ visuel de l'utilisateur (110, 220), et une image de position cible représentant la position de l'objet cible de rééducation.

2. L'appareil (210) d'aide à la rééducation selon la revendication 1, dans lequel le deuxième contrôleur d'affichage (103, 213) est configuré pour provoquer l'affichage de l'image de notification (131) au centre de l'écran d'affichage du casque de réalité virtuelle (111, 233), indépendamment de la direction de la tête de l'utilisateur (110, 220).

3. L'appareil (210) d'aide à la rééducation selon l'une quelconque des revendications 1 à 2, dans lequel l'image de notification (131) comprend une image de tête représentant la direction de la tête de l'utilisateur (110, 220) vue de dessus, et l'image de position cible représentant la position de l'objet cible de rééducation.

4. L'appareil (210) d'aide à la rééducation selon l'une quelconque des revendications 1 à 3, dans lequel l'image de notification (131) comprend une image de bloc générée en divisant une périphérie de l'utilisateur (110, 220) en une pluralité de blocs.

5. L'appareil (210) d'aide à la rééducation selon l'une quelconque des revendications 1 à 4, comprenant en outre une unité de commande (217) utilisée par l'opérateur pour commander l'affichage par le premier contrôleur d'affichage (102, 212) et le deuxième contrôleur d'affichage (103, 213),
l'unité de commande (217) étant configurée pour afficher, à l'intention de l'opérateur, un écran de commande (300).

6. L'appareil (210) d'aide à la rééducation selon la revendication 5, dans lequel l'unité de commande est configurée pour accepter, à partir du bouton de recentrage (305), qui est un bouton qui accepte, de l'opérateur, une instruction de reconstruction pour reconstruire l'espace virtuel en fonction de la position de l'utilisateur (110, 220), et conformément à l'instruction de reconstruction, le premier contrôleur d'affichage (102, 212) reconstruit l'espace virtuel dans lequel la position du casque de réalité virtuelle (111, 233) à l'instant est définie comme origine, et la direction du casque de réalité virtuelle (111, 233) à l'instant est définie comme direction de référence.

7. Un système d'aide à la rééducation (200) comprenant :
un appareil (210) d'aide à la rééducation selon l'une quelconque des revendications 1 à 6 ;
un casque de réalité virtuelle (111, 233) ;
un capteur, configuré pour détecter la position de la main d'un utilisateur (110, 220) ; et
un évaluateur (214), qui est configuré pour comparer la position de la main de l'utilisateur (110, 220), capturée par le capteur, avec une position cible représentée par un objet cible de rééducation, et pour évaluer la capacité de l'utilisateur (110, 220).

8. Un procédé d'aide à la rééducation mis en œuvre par un appareil (210) d'aide à la rééducation comprenant un détecteur (101, 211), un premier contrôleur d'affichage (102, 212) et un deuxième contrôleur d'affichage (103, 213), le procédé d'aide à la rééducation comprenant :
le fait, par le détecteur (101, 211), de détecter une direction de la tête d'un utilisateur (110, 220) portant un casque de réalité virtuelle (111, 233) ;
le fait, par le premier contrôleur d'affichage (102, 212), dans un espace virtuel, de générer un objet cible de rééducation (122, 242) destiné à être reconnu visuellement par l'utilisateur (110, 220) et d'afficher l'objet cible de rééducation sur un écran d'affichage (340) du casque de réalité virtuelle (111, 233) conformément à la direction de la tête de l'utilisateur détectée lors de la détection, le premier contrôleur d'affichage (102, 212) affichant l'objet cible de rééducation (122, 242) tout en modifiant progressivement la position et la taille d'affichage de l'objet cible de rééducation (122) de manière à ce qu'il tombe vers le bas depuis au-dessus de l'utilisateur (220) ; et
le fait, par le deuxième contrôleur d'affichage (103, 213), d'afficher sur l'écran d'affichage (340) du casque de réalité virtuelle (111, 233) une image de notification (131) utilisée pour notifier à l'utilisateur (110, 220) la position d'affichage de l'objet cible de rééducation ;
le deuxième contrôleur d'affichage (103, 213) est configuré pour provoquer l'affichage d'une direction de référence (311) dans l'espace virtuel sur l'écran d'affichage (340) du casque de réalité virtuelle (111, 233), la direction de référence indiquant la direction de la tête de l'utilisateur (110, 220) portant le casque de réalité virtuelle (111, 233) lorsqu'un bouton de recentrage est actionné par un opérateur ;
l'image de notification (131) est une image d'écran radar (250) qui est configurée pour montrer une direction relative de la position de l'objet cible de rééducation par rapport à une direction de référence dans l'espace virtuel ;
l'image de notification (131) est l'image d'écran radar qui est configurée pour montrer une direction relative de la position de l'objet cible de rééducation par rapport à la direction de la tête de l'utilisateur (110, 220) ; et
l'image d'écran radar (250) comprend une image de zone de champ visuel (253) représentant un champ visuel de l'utilisateur (110, 220), et une image de position cible représentant la position de l'objet cible de rééducation.

9. Un programme d'aide à la rééducation comprenant une pluralité d'instructions lisibles par ordinateur qui, lorsqu'elles sont exécutées par un ordinateur, amènent l'ordinateur à mettre en oeuvre les étapes du procédé selon la revendication 8.
